# EUROPÄISCHE PATENTSCHRIFT

(11) **EP 1 218 055 B1**
(45) Veröffentlichungstag und Bekanntmachung des Hinweises auf die Patenterteilung: **03.12.2003**
(21) Anmeldenummer: 00972597.9
(22) Anmeldetag: 20.09.2000
(51) Int. Cl.: A61M 39/00

(54) **SCHLAUCHKLEMME, INFUSIONSPUMPE, INFUSIONSSCHLAUCHSET, KOMBINATION EINER INFUSIONSPUMPE MIT EINEM INFUSIONSSCHLAUCHSET**
TUBE CLAMP, INFUSION PUMP, INFUSION TUBE SET, COMBINATION OF INFUSION PUMP WITH INFUSION TUBE SET
CLAMP POUR TUYAU FLEXIBLE, POMPE A PERFUSION, ENSEMBLE TUYAU FLEXIBLE DE PERFUSION, ET COMBINAISON D'UNE POMPE A PERFUSION ET D'UN ENSEMBLE TUYAU FLEXIBLE DE PERFUSION

(30) Priorität: 05.10.1999 DE 19947973
(43) Veröffentlichungstag der Anmeldung: 03.07.2002
(73) Patentinhaber: Fresenius AG, 61352 Bad Homburg (DE)
(72) Erfinder: ZOLTAN, Hubert, F-38340 La Placette (FR); PONCON, Gilbert, F-38340 Pommiers la Placette (FR); WILMERS, Andreas, 61169 Friedberg (DE); PLAZY, Jean-Marc, F-38240 Meylan (FR)
(74) Vertreter: Vièl, Christof, Dipl.-Ing.
(86) Internationale Anmeldenummer: DE0003282
(87) Internationale Veröffentlichungsnummer: WO01024870

(56) Entgegenhaltungen:
- EP-A- 0 637 456
- WO-A-96/00598
- WO-A-99/18377
- US-A- 5 951 519

## Beschreibung

Die Erfindung betrifft die Kombination einer Infusionspumpe mit einem Infusionsschlauchset, wobei der Schlauch durch eine Schlauchklemme geführt wird, mit der der Schlauch wahlweise geöffnet un/oder abgesperrt werden kann, sowie eine Schlauchklemme zum wahlweisen Absperren und Öffnen von Schläuchen, z.B. Infusionsschläuchen, in Form eines im wesentlichen U-förmigen Bügels mit steifen Schenkeln, auf deren Innenseite als Quetschkanten zusammenwirkende Vorsprünge ausgebildet sind, einem biegeelastischen Steg, durch welchen die Schenkel in eine divergierende Stellung aufspreizbar sind, und einer mit dem freien Ende des ersten Schenkels verbundenen, zum zweiten Schenkel hin gebogenen, biegeelastisch auslenkbaren Federzunge mit einer einwärts weisenden Rastnase, hinter welcher beim Zusammendrücken der Schenkel das freie Ende des zweiten Schenkels in der Absperrstellung einrastbar ist, wobei der Steg und die Federzunge mit Löchern zum Hindurchführen des Schlauches versehen sind. Sie betrifft auch eine Infusionspumpe, die eine ver- und entriegelbare Tür zum Einlegen und Entnehmen eines durch eine Schlauchklemme geführten Schlauches aufweist, wobei die Schlauchklemme wahlweise geöffnet und/oder abgesperrt werden kann bzw. in der Absperrstellung einrastbar ist. Weiterhin betrifft die Erfindung ein Infusionsschlauchset, wobei ein Schlauch durch eine Schlauchklemme geführt wird, mit der der Schlauch wahlweise geöffnet und/oder abgesperrt werden kann.

Eine Schlauchklemme, wie sie oben beschrieben wurde, ist beispielsweise aus der EP 0 637 456 A1 bekannt. Derartige Schlauchklemmen sitzen in der offenen Stellung lose auf dem Schlauch und behindern den Durchfluß nicht. Drückt man die Schenkel bis zum Einrasten der Federzunge zusammen, behält die Schlauchklemme eine Absperrstellung, in welcher der Schlauch durch die inneren Vorsprünge der Schenkel dicht zusammengequetscht wird. Die Absperrstellung läßt sich leicht mit einer Hand lösen, indem die Federzunge mit Rastnase nach außen zurückgebogen wird, so daß sie den von ihr übergriffenen Schenkel wieder freigibt.

Diese Schlauchklemmen werden auch in Infusionssets verwendet, wie sie oben beschrieben wurden.

Aus der US 5,817,116 A ist eine Vorrichtung zum Lösen eines Schlauches für medizinische Behandlungen bekannt, die ein schnelles Lösen der Vorrichtung von dem Patienten in Notfällen, wie z.B. Erdbeben oder Feuer, ermöglicht. Durch die Vorrichtung wird ein mit dem Körper des Patienten verbundener medizinischer Schlauch an zwei Stellen verschlossen und der Schlauch zwischen diesen beiden Stellen durchtrennt.

In der EP 0 767 691 B1 wird ein Infusionsset beschrieben, das verschiebbare Absperrmittel aufweist, die eine Öffnung für den Schlauch aufweisen. Hierbei ist vorgesehen, daß in einer ersten Position der Schlauch durch einen Bereich der Öffnung hindurchtritt, welche genügend große Abmessungen aufweist, um ein Fließen des Infusionsmittels durch den Schlauch zu ermöglichen, und in der zweiten Position der Schlauch durch einen Bereich der Öffnung mit wesentlich kleineren Abmessungen als dem Schlauchdurchmesser durchtritt, so daß der Schlauch dann flüssigkeitsdicht abgeschlossen ist. Es sind eine Zunge und ein Rasthaken vorgesehen, die so angeordnet sind, daß die Absperrmittel beim Öffnen des Infusionssets von der ersten zur zweiten Position verschoben werden und beim Schließen von der zweiten zu der ersten Position.

Allerdings ist bei dieser Ausführung von Nachteil, daß der Schlauch und die Absperrmittel eine Relativbewegung zueinander ausführen, was zu einem Verklemmen des Schlauches fuhren kann. Zudem ist die Abnutzung des Schlauches aufgrund der Relativbewegung von Nachteil.

Aufgabe der Erfindung ist es somit, eine Schlauchklemme zu schaffen, bei der ohne Relativbewegung zwischen der Schlauchklemme und dem Schlauch ein automatisiertes Verschließen und Öffnen der Schlauchklemme möglich ist. Weiterhin sollen eine hierfür geeignete Infusionspumpe und ein Infusionsschlauchset geschaffen werden, in die die erfindungsgemäße Schlauchklemme integriert werden kann.

Diese Aufgabe wird bei einer Kombination einer Infusionspumpe mit einem Infusionsschlauchset, wobei der Schlauch durch eine Schlauchklemme geführt wird, mit der der Schlauch wahlweise geöffnet und/oder abgesperrt werden kann, erfindungsgemäß dadurch gelöst, daß
- eine Infusionspumpe, die eine ver- und entriegelbare Tür zum Einlegen und Entnehmen eines durch eine Schlauchklemme geführten Schlauches aufweist, wobei die Schlauchklemme wahlweise geöffnet und/oder abgesperrt werden kann bzw. in der Absperrstellung einrastbar ist, wobei die Infusionspumpe ein Wirkglied zum Schließen und/oder Öffnen einer Schlauchklemme aufweist, die jeweils beim Zusammenwirken mit einem Teil der Schlauchklemme diese öffnet und/oder schließt,
- und ein Infusionsschlauchset, wobei ein Schlauch durch eine Schlauchklemme geführt wird, mit der der Schlauch wahlweise geöffnet und/oder abgesperrt werden kann,
verwendet werden,
- wobei die Schlauchklemme zum wahlweisen Absperren und Öffnen von Schläuchen, z.B. Infusionsschläuchen, dient und in Form eines im wesentlichen U-förmigen Bügels mit steifen Schenkeln ausgebildet ist, auf deren Innenseite als Quetschkanten zusammenwirkende Vorsprünge ausgebildet sind, mit einem biegeelastischen Steg, durch welchen die Schenkel
- in eine divergierende Stellung aufspreizbar sind, und einer mit dem freien Ende des ersten Schenkels verbundenen, zum zweiten Schenkel hin gebogenen, biegeelastisch auslenkbaren Federzunge mit einer einwärts weisenden Rastnase, hinter welcher beim Zusammendrücken der Schenkel das freie Ende des zweiten Schenkels in der Absperrstellung einrastbar ist, wobei der Steg und die Federzunge mit Löchern zum Hindurchführen des Schlauches versehen sind, und wobei die Schlauchklemme Mittel zum Schließen der Schlauchklemme und/oder Mittel zum Öffnen der Schlauchklemme aufweist, die jeweils bei Druckausübung durch das Wirkglied der Infusionspumpe die Schlauchklemme öffnen und/oder schließen.

Die erfindungsgemäße Schlauchklemme ist dadurch gekennzeichnet, daß die Schlauchklemme Mittel zum Schließen der Schlauchklemme und/oder Mittel zum Öffnen der Schlauchklemme aufweist, die jeweils bei Druckausübung durch ein Wirkglied einer Infusionspumpe die Schlauchklemme öffnen und/oder schließen.

Erfindungsgemäß ist vorgesehen, daß die Mittel zum Schließen der Schlauchklemme als Element an der Schlauchklemme ausgebildet sind, auf die ein Teil der Pumpe eine vertikal auf den zweiten Schenkel der Schlauchklemme wirkende Kraft ausüben kann.

Eine Ausbildung der Erfindung besteht darin, daß die Mittel zum Schließen der Schlauchklemme als Auswölbung an der Außenseite des zweiten Schenkels ausgebildet sind, auf der sich zumindest ein Teil eines Kipphebels der Infusionspumpe abrollen kann.

Der Erfindung liegt die Erkenntnis zugrunde, daß beispielsweise der ohnehin vorhandene Kipphebel zum Ver- und Entriegeln der Tür auch zum Öffnen und Verschließen des Schlauches verwendet werden kann. Durch eine entsprechende Anpassung der Schlauchklemme kann diese mit dem Kipphebel zusammenwirken.

Während des Entriegelns der Tür führt der Kipphebel eine Drehbewegung aus und im Zuge dieser Drehbewegung kann das Ende des Kipphebels auf der Auswölbung an der Außenseite des zweiten Schenkels der Schlauchklemme abrollen und diese in die Schließstellung bringen. Bei dem Verriegeln der Tür erfolgt dann der umgekehrte Vorgang.

Eine Weiterbildung der Erfindung besteht darin, daß die Mittel zum Schließen der Schlauchklemme eine Verzahnung aufweisen, die mit einer entsprechenden Verzahnung des Kipphebels zusammenwirken kann.

Hierdurch wird ein Schlupf zwischen den auf dem Schenkel der Schlauchklemme angeordneten Mitteln zum Zusammenwirken mit dem Kipphebel und dem Kipphebel selbst vermieden.

Es liegt im Rahmen der Erfindung, daß ein in die Schlauchklemme eingelegter Schlauch durch das Zusammenwirken der Mittel zum Schließen der Schlauchklemme mit dem Kipphebel flüssigkeitsdicht absperrbar ist.

Ebenfalls ist es zweckmäßig, daß die Schlauchklemme durch das Zusammenwirken der Mittel zum Schließen der Schlauchklemme mit dem Kipphebel einrastbar ist.

Es liegt im Rahmen der Erfindung, daß die Mittel zum Öffnen der Schlauchklemme als Element an der Schlauchklemme ausgebildet sind, über das ein Teil der Pumpe die Federzunge von dem zweiten Schenkel weg drücken kann.

Weiterhin es erfindungsgemäß vorgesehen, daß die Mittel zum Öffnen der Schlauchklemme an der Federzunge als Element zum Zusammenwirken mit zumindest einem Teil eines Kipphebels der Infusionspumpe ausgebildet ist, wobei die Federzunge von dem zweiten Schenkel weg gedrückt wird.

Im Rahmen der Erfindung liegt auch eine Infusionspumpe mit einem Infusionsschlauchset, wobei die Infusionspumpe eine durch einen Kipphebel ver- und entriegelbare Tür zum Einlegen und Entnehmen eines Schlauches aufweist und wobei der Schlauch durch eine Schlauchklemme geführt wird, mit der der Schlauch wahlweise geöffnet und/oder abgesperrt werden kann bzw. in der Absperrstellung einrastbar ist, wobei die Infusionspumpe ein Wirkglied zum Schließen und/oder Öffnen einer Schlauchklemme aufweist, das jeweils durch Druckausübung auf Mittel zum Schließen und/oder Mittel zum Öffnen der Schlauchklemme die Schlauchklemme öffnet und/oder schließt.

Im Rahmen der Erfindung liegt auch die Verwendung eines Infusionsschlauchsets, wobei ein Schlauch durch eine Schlauchklemme geführt wird, mit der der Schlauch wahlweise geöffnet und/oder abgesperrt werden kann bzw. in der Absperrstellung einrastbar ist in einer Infusionspumpe.

Die Vorteile der Erfindung liegen im wesentlichen darin, daß ohne Relativbewegung zwischen der Schlauchklemme und dem Schlauch durch den ohnehin vorhandenen Hebel eine Freigabe und ein Verschließen des Schlauches möglich sind, die im Einklang mit dem Ver- bzw. Entriegeln der Tür stehen.

Im folgenden wird die Erfindung anhand von Ausführungsbeispielen erläutert,

Es zeigen
- Fig. 1: eine erfindungsgemäße Schlauchklemme,
- Fig. 2a bis Fig. 2 d: das Verschließen einer erfindungsgemäßen Schlauchklemme mittels eines Kipphebels,
- Fig. 3a bis Fig. 3c,: eine andere Ausführung einer erfindungsgemäßen Schlauchklemme in Seitenansicht, geschnittener Darstellung und Draufsicht.

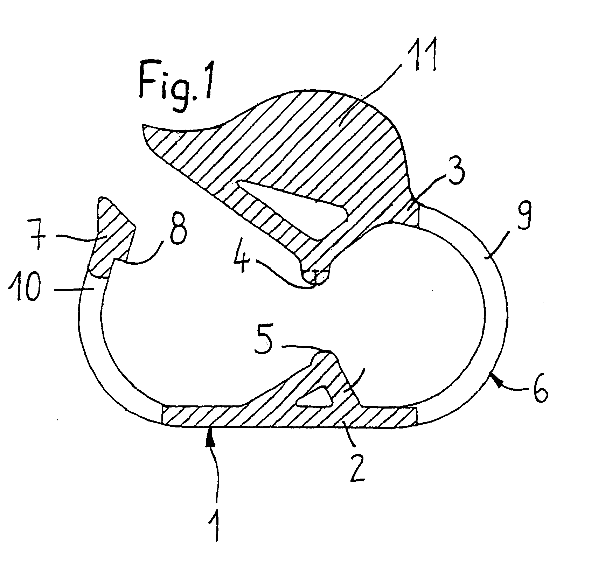

Wie aus den Fig. 1 hervorgeht, weist die Schlauchklemme 1 einen ersten Schenkel 2 und einen zweiten Schenkel 3 auf, auf deren Innenseiten als Quetschkanten zusammenwirkende Vorsprünge 4, 5 ausgebildet sind und die durch einen biegeelastischen Steg 6, durch welchen die Schenkel 2, 3 in eine divergierende Stellung aufspreizbar sind, verbunden sind.

Eine mit dem freien Ende des ersten Schenkels 2 verbundene, zum zweiten Schenkel 3 hin gebogene, biegeelastische Federzunge 7 mit einer einwärts weisenden Rastnase 8, hinter welcher beim Zusammendrücken der Schenkel 2, 3 das freie Ende des zweiten Schenkels 3 in der Absperrstellung einrastbar ist, dient zum Halten der Schlauchklemme 1 in Schließstellung. Der Steg 6 und die Federzunge 7 sind mit Löchern 9, 10 zum Hindurchführen eines Schlauches versehen. Der zweite Schenkel 3 weist an seiner Außenseite Mittel 11 zum Schließen der Schlauchklemme auf, die im vorliegenden Fall, der Form des Kipphebels entsprechend, als Auswölbung ausgebildet sind, auf der das Ende 14 des Kipphebels 12 abrollen kann.

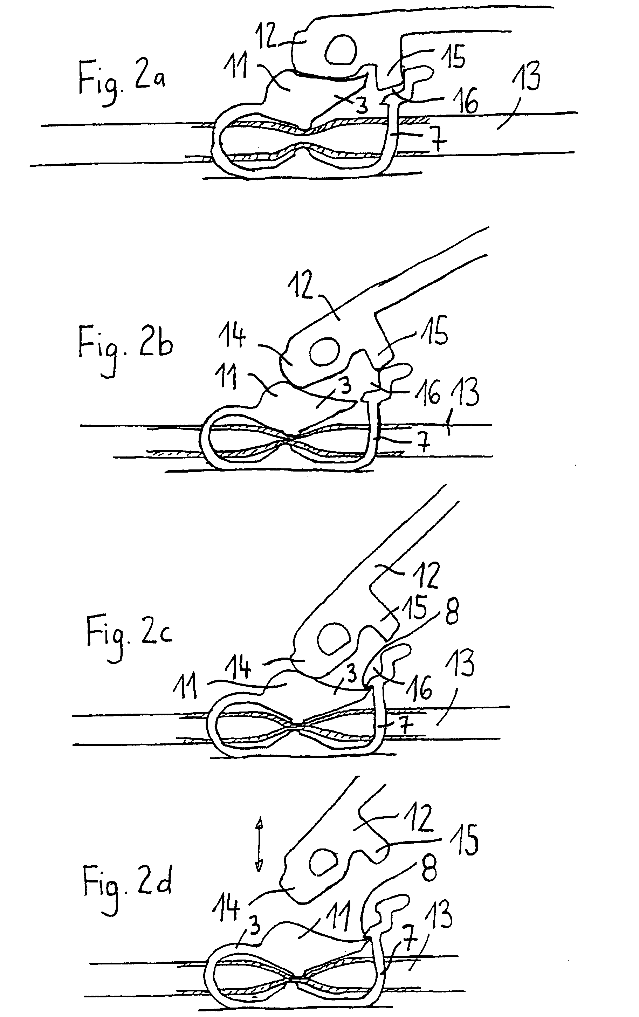

Die Fig. 2a bis 2d beschreiben das Zusammenwirken von Kipphebel 12 und den Mitteln 11 zum Schließen der Schlauchklemme sowie den Mitteln 16 zum Öffnen der Schlauchklemme. In der Ausgangsstellung (nach Fig. 2a) liegt der Ansatz 15 des Kipphebels 12 auf dem dafür vorgesehenen als Absatz ausgebildeten Element 16 der Schlauchklemme 1, das in etwa rechtwinklig zur Federzunge 7 angeordnet ist, auf und der Schlauch 13 durchläuft die Schlauchklemme 1, ohne daß die Vorsprünge 4,5 einen bemerkenswerten Druck auf den Schlauch 13 ausübten. Die Flüssigkeit kann in dem Schlauch 13 zirkulieren.

Ausgehend von der in Fig. 2a gezeigten Stellung beginnt der Kipphebel 12 seine Drehbewegung und übt mit seinem Ende 14 Druck auf die Mittel 11 zum Zusammenwirken mit dem Kipphebel 12, hier die Auswölbung 11, aus. Die Vorsprünge 4, 5 beginnen, den Schlauch 13 zusammenzuquetschen.

In Fig. 2b hat der Kipphebel 12 bereits mit seinem Ende 14 den Schlauch 12 so stark zusammengedrückt, daß dieser flüssigkeitsdicht zusammengequetscht ist. Aufgrund der Abrollbewegung des Endes 14 des Kipphebels 12 auf der Auswölbung 11 wird der zweite Schenkel 3 der Schlauchklemme 1 auf die Federzunge 7 hin bewegt und nach unten gedrückt. Gleichzeitig federt der Steg 6 in seine Ausgangsstellung, da der Ansatz 15 des Kipphebels 12 nicht mehr auf das Element 16 drückt.

Fig. 2c zeigt den Moment des Einrastens des zweiten Schenkels 3 in der Rastnase 8 der Federzunge 7, nachdem der Kipphebel 12 sich weiter gedreht hat.

Wie in Fig. 2d dargestellt ist, kann nach dem Verschließen der Schlauchklemme 1 der Kipphebel 12 angehoben werden.

In aller Regel wird das Schlauchset aus Lagerungsgründen mit der Schlauchklemme in geöffnetem Zustand ausgeliefert. Vor der Benutzung schließt der Anwender die Schlauchklemme und legt das Schlauchset in die Pumpe ein. Die Pumpe muß nun in der Lage sein, die Schlauchklemme zu öffnen. Dieses Öffnen der Schlauchklemme 1 erfolgt in umgekehrter Reihenfolge zu der in den Fig. 2a bis 2d gezeigten. Zum Öffnen wird durch Krafteinwirkung in etwa parallel zur Federzunge 7 auf das als Absatz ausgebildete Element 16 wird ein Drehmoment erzeugt, das zur Aufweitung der Schlauchklemme führt.

Hierbei sei bemerkt, daß statt des Absatzes 16 auch jedes andere Element zum Zusammenwirken mit dem Kipphebel 12 an der Schlauchklemme vorliegen könnte, sofern durch dieses Zusammenwirken die Federzunge 7 von dem zweiten Schenkel 3 der Schlauchklemme weggedrückt werden kann. Ebenso kommt es beim Schließen lediglich darauf an, daß Druck in vertikaler Richtung auf den zweiten Schenkel 3 der Schlauchklemme ausgeübt wird.

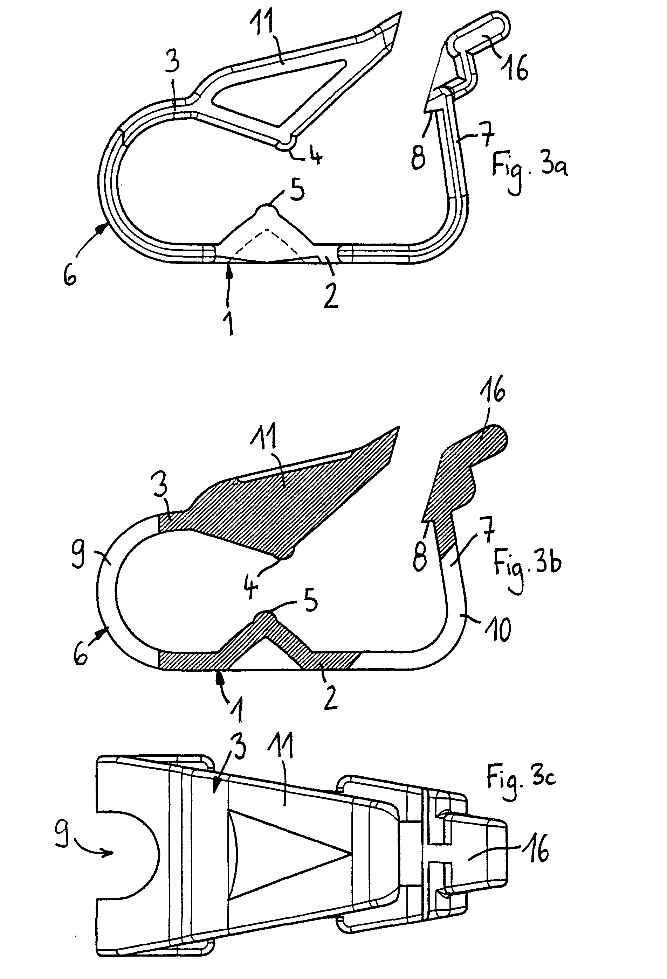

Wie aus den Fig. 3a bis 3d ersichtlich ist, kann die erfindungsgemäße Schlauchklemme auch so ausgebildet sein, daß statt des als Absatz ausgebildeten Elementes 16 eine Schräge vorliegt, an der beim Schließen das Ende des zweiten Schenkels 3 entlangläuft, bis er unter der Rastnase 8 der Federzunge 7 einrastet. Bei dieser Ausmhrungsform kommt das Gegenstück 15 auf Seiten der Infusionspumpe nur noch mit dem oberen Absatz 16 in Berührung. Durch Krafteinwirkung in etwa parallel zu der Federzunge 7 auf dieses als Absatz ausgebildete Element 16 wird beim Öffnen der Schlauchklemme ein Drehmoment erzeugt, das zur Aufweitung der Schlauchklemme führt.

Es kann erforderlich sein, daß bei der Abrollbewegung des Kipphebels 12 auf den Mitteln 11 bzw. 16 zum Schließen bzw. Öffnen der Schlauchklemme ein möglichst geringer Schlupf zwischen diesen besteht. Dies kann beispielsweise dadurch realisiert werden, daß die zusammenwirkenden Flächen des Kipphebels 12 und der Mittel 11 bzw. 16 mit die Reibung erhöhenden Auflagen versehen sind oder daß eine Verzahnung zwischen diesen vorgesehen ist.

Es kann bei einer derartigen, automatisch durch die Infusionspumpe betätigbaren Schlauchklemme zweckmäßig sein, Mittel zur Erkennung des Vorhandenseins einer Schlauchklemme in der Pumpe vorzusehen, beispielsweise ein optisches Erkennungssystem oder ein elektrisches Erkennungssystem (z.B. durch Verwendung elektrisch leitenden Kunststoffs für die Schlauchklemme).

## Patentansprüche

1. Kombination einer Infusionspumpe mit einem Infusionsschlauchset, wobei der Schlauch durch eine Schlauchklemme geführt wird, mit der der Schlauch wahlweise geöffnet und/oder abgesperrt werden kann, **dadurch gekennzeichnet, daß**
• eine Infusionspumpe, die eine ver- und entriegelbare Tür zum Einlegen und Entnehmen eines durch eine Schlauchklemme (1) geführten Schlauches (13) aufweist, wobei die Schlauchklemme (1) wahlweise geöffnet und/oder abgesperrt werden kann bzw. in der Absperrstellung einrastbar ist, wobei die Infusionspumpe ein Wirkglied (12) zum Schließen und/oder Öffnen einer Schlauchklemme (1) aufweist, die jeweils beim Zusammenwirken mit einem Teil der Schlauchklemme (1) diese öffnet und/oder schließt,
• und ein Infusionsschlauchset, wobei ein Schlauch (13) durch eine Schlauchklemme (1) geführt wird, mit der der Schlauch (13) wahlweise geöffnet und/oder abgesperrt werden kann,
verwendet werden,
• wobei die Schlauchklemme (1) zum wahlweisen Absperren und Öffnen von Schläuchen (13), z.B. Infusionsschläuchen, dient und in Form eines im wesentlichen U-förmigen Bügels mit steifen Schenkeln (2, 3) ausgebildet ist, auf deren Innenseite als Quetschkanten zusammenwirkende Vorsprünge (4, 5) ausgebildet sind, mit einem biegeelastischen Steg (6), durch welchen die Schenkel (2, 3) in eine divergierende Stellung aufspreizbar sind, und einer mit dem freien Ende des ersten Schenkels (2) verbundenen, zum zweiten Schenkel (3) hin gebogenen, biegeelastisch auslenkbaren Federzunge (7) mit einer einwärts weisenden Rastnase (8), hinter welcher beim Zusammendrücken der Schenkel (2, 3) das freie Ende des zweiten Schenkels (3) in der Absperrstellung einrastbar ist, wobei der Steg (6) und die Federzunge (7) mit Löchern (9, 10) zum Hindurchführen des Schlauches (13) versehen sind, und wobei die Schlauchklemme (1) Mittel (11) zum Schließen der Schlauchklemme (1) und/oder Mittel (16) zum Öffnen der Schlauchklemme (1) aufweist, die jeweils bei Druckausübung durch das Wirkglied einer Infusionspumpe die Schlauchklemme (1) öffnen und/oder schließen.

2. Schlauchklemme zur Verwendung in einer Kombination gemäß Anspruch 1, zum wahlweisen Absperren und Öffnen von Schläuchen, z.B. Infusionsschläuchen, in Form eines im wesentlichen U-förmigen Bügels mit steifen Schenkeln, auf deren Innenseite als Quetschkanten zusammenwirkende Vorsprünge ausgebildet sind, einem biegeelastischen Steg, durch welchen die Schenkel in eine divergierende Stellung aufspreizbar sind, und einer mit dem freien Ende des ersten Schenkels verbundenen, zum zweiten Schenkel hin gebogenen, biegeelastisch auslenkbaren Federzunge mit einer einwärts weisenden Rastnase, hinter welcher beim Zusammendrücken der Schenkel das freie Ende des zweiten Schenkels in der Absperrstellung einrastbar ist, wobei der Steg und die Federzunge mit Löchern zum Hindurchführen des Schlauches versehen sind, **dadurch gekennzeichnet, daß** die Schlauchklemme (1) Mittel (11) zum Schließen der Schlauchklemme und/oder Mittel (16) zum Öffnen der Schlauchklemme (1) aufweist, die jeweils bei Druckausübung durch ein Wirkglied einer Infusionspumpe die Schlauchklemme (1) öffnen und/oder schließen.

3. Schlauchklemme gemäß Anspruch 2, **dadurch gekennzeichnet, daß** die Mittel (11) zum Schließen der Schlauchklemme (1) als Element (11) an der Schlauchklemme (1) ausgebildet sind, auf die ein Teil der Infusionspumpe eine vertikal auf den zweiten Schenkel (3) der Schlauchklemme (1) wirkende Kraft ausüben kann.

4. Schlauchklemme gemäß Anspruch 3, **dadurch gekennzeichnet, daß** die Mittel (11) zum Schließen der Schlauchklemme (1) als Auswölbung (11) an der Außenseite des zweiten Schenkels (3) ausgebildet sind, auf der sich zumindest ein Teil (14) eines Kipphebels (12) der Infusionspumpe abrollen kann.

5. Schlauchklemme gemäß Anspruch 4, **dadurch gekennzeichnet, daß** die Mittel (11) zum Schließen der Schlauchklemme (1) eine Verzahnung aufweisen, die mit einer entsprechenden Verzahnung des Kipphebels (12) zusammenwirken kann.

6. Schlauchklemme gemäß einem der Ansprüche 2 bis 5, **dadurch gekennzeichnet, daß** ein in die Schlauchklemme (1) eingelegter Schlauch (13) durch das Zusammenwirken der Mittel (11) zum Schließen der Schlauchklemme (1) mit dem Kipphebel (12) flüssigkeitsdicht absperrbar ist.

7. Schlauchklemme gemäß einem der Ansprüche 2 bis 5, **dadurch gekennzeichnet, daß** die Schlauchklemme (1) durch das Zusammenwirken der Mittel ( 11 ) zum Schließen der Schlauchklemme (1) mit dem Kipphebel (12) einrastbar ist.

8. Schlauchklemme gemäß Anspruch 2, **dadurch gekennzeichnet, daß** die Mittel (16) zum Öffnen der Schlauchklemme (1) als Element (16) an der Schlauchklemme (1) ausgebildet sind, über das ein Teil der Infusionspumpe die Federzunge (7) von dem zweiten Schenkel (3) weg drücken kann.

9. Schlauchklemme gemäß Anspruch 8, **dadurch gekennzeichnet, daß** die Mittel (16) zum Öffnen der Schlauchklemme (1) an der Federzunge (7) als Element (16) zum Zusammenwirken mit zumindest einem Teil (15) des Kipphebels (12) der Infusionspumpe ausgebildet ist, wobei die Federzunge (7) von dem zweiten Schenkel (3) weg gedrückt wird.

10. Infusionspumpe zur Verwendung in einer Kombination gemäß Anspruch 1, die eine ver- und entriegelbare Tür zum Einlegen und Entnehmen eines durch eine Schlauchklemme geführten Schlauches aufweist, wobei die Schlauchklemme wahlweise geöffnet und/oder abgesperrt werden kann bzw. in der Absperrstellung einrastbar ist, **dadurch gekennzeichnet, daß** die Infusionspumpe ein Wirkglied (12) zum Schließen und/oder Öffnen einer Schlauchklemme (1) gemäß den Ansprüchen 2 bis 9 aufweist, das jeweils durch Druckausübung auf Mittel (11) zum Schließen und/oder Mittel (16) zum Öffnen der Schlauchklemme (1) die Schlauchklemme (1) öffnet und/oder schließt.

11. Infusionsschlauchset, wobei ein Schlauch durch eine Schlauchklemme gemäß den Ansprüchen 2 bis 9 geführt wird, mit der der Schlauch wahlweise geöffnet und/oder abgesperrt werden kann zur Verwendung in einer Infusionspumpe gemäß Anspruch 10.

## Claims

1. A combination of an infusion pump and an infusion tube set, the tube being guided through a tube clamp with which the tube can be selectively opened and/or closed, **characterized in that**
• an infusion pump having a lockable and unlockable door for insertion and removal of a tube (13) guided through a tube clamp (1) that can be selectively opened and/or closed and is engageable in the locking position, the infusion pump being provided with an actuating member (12) for closing and/or opening a tube clamp (1) and being configured to interact with a part of the tube clamp (1) to thereby open and/or close said tube clamp,
• and an infusion tube set, the tube (13) being guided through a tube clamp (1) with which the tube (13) can be selectively opened and/or closed,
are used,
• the tube clamp (1) serving for the selective closing and opening of tubes (13), for example infusion tubes, and being configured as an essentially U-shaped shackle, said shackle having rigid legs (2,3) provided with inside projections (4,5) that interact with each other as pinch-off edges and are connected by a flexible web (6) by means of which the legs (2,3) can be spread into a diverging position, said first leg (2) being connected at its free end with a flexible spring latch (7) that curves towards the second leg (3) and is provided with an inward-pointing cam (8), behind which the free end of the second leg (3) engages in the locking position when the legs (2,3) are pressed together, the web (6) and the spring latch (7) being provided with openings (9,10) for guiding the tube (13) therethrough, and the tube clamp (1) being provided with means (11) for closing the tube clamp (1) and/or means (16) for opening the tube clamp (1), which open and/or close the tube clamp (1) in response to pressure exerted by the actuating member of an infusion pump.

2. A tube clamp for use in the combination of claim 1, for selectively closing and opening tubes such as infusion tubes, being configured as an essentially U-shaped shackle having rigid legs provided with inside projections that interact with each other as pinch-off edges, a flexible web by means of which the legs can be spread into a diverging position and a flexible spring latch connected to the free end of the first leg and curving towards the second leg, said latch having an inward-pointing cam behind which the free end of the second leg engages in the locking position when the legs are pressed together, the web and the spring latch being provided with openings for guiding the tube therethrough, **characterized in that** the tube clamp (1) has means (11) for closing the tube clamp and/or means (16) for opening the tube clamp (1), which open and/or close the tube clamp (1 ) in response to pressure exerted by an actuating member of an infusion pump.

3. The tube clamp of claim 2, **characterized in that** the means (11 ) for closing the tube clamp (1) is configured as an element (11) on the tube clamp (1) onto which a component of the infusion pump can exert a force which acts in a vertical direction on the second leg (3) of the tube clamp (1 ).

4. The tube clamp of claim 3, **characterized in that** the means (11 ) for closing the tube clamp (1) is configured as an arched portion (11) on the outer surface of the second leg (3), on which at least a portion (14) of a rocking lever (12) of the infusion pump can interact.

5. The tube clamp of claim 4, **characterized in that** the means (11) for closing the tube clamp (1) has a toothed surface for interacting with a complementary toothed surface of the rocking lever (12).

6. The tube clamp according to one of the claims 2 to 5, **characterized in that** a tube (13) inserted into the tube clamp (1) can be clamped shut in liquid-tight manner by interaction of the means (11) for closing the tube clamp (1) with the rocking lever (12).

7. The tube clamp according to one of the claims 2 to 5, **characterized in that** the tube clamp (1) engages in the locking position when the means (11) for closing the tube clamp (1) interacts with the rocking lever (12).

8. The tube clamp of claim 2, **characterized in that** the means (16) for opening the tube clamp (1) is configured as an element (16) on the tube clamp (1), by way of which a component of the infusion pump can move the spring latch (7) away from the second leg (3).

9. The tube clamp of claim 8, **characterized in that** the means (16) for opening the tube clamp (1) at the spring latch (7) is configured as an element (16) for interacting with at least a portion (15) of the rocking lever (12) of the infusion pump, with the spring latch (7) being moved away from the second leg (3).

10. An infusion pump for use in the combination of claim 1, having a lockable and unlockable door for insertion and removal of a tube guided through a tube clamp that can be selectively opened and/or closed and is engageable in the locking position, **characterized in that** the infusion pump is provided with an actuating member (12) for closing and/or opening a tube clamp (1) of the kind claimed in claims 2 to 9, said actuating member opening and/or closing the tube clamp (1) by means of exerting pressure on the means (11) for closing and/or means (16) for opening the tube clamp (1).

11. An infusion tube set comprising a tube guided through the tube clamp of claims 2 to 9, with which clamp the tube can be selectively opened and/or closed, for use in the infusion pump of claim 10.

## Revendications

1. Combinaison d'une pompe pour perfusion et d'un jeu de tuyau flexible pour la perfusion, le tuyau flexible étant passé à travers une pince à tuyau flexible avec laquelle le tuyau flexible peut être au choix ouvert ou fermé, **caractérisée en ce que**
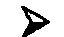 une pompe pour perfusion, munie d'une porte pouvant être verrouillée ou déverrouillée pour mettre en place ou enlever un tuyau flexible (13) passant à travers une pince à tuyau flexible (1), la pince à tuyau flexible (1) pouvant être au choix ouverte et/ou verrouillée ou bien être encliquetable en position verrouillée, la pompe pour perfusion étant munie pour fermer et/ou ouvrir une pince à tuyau flexible (1) d'un élément d'actionnement (12) qui ouvre et/ou ferme la pince à tuyau flexible (1) à chaque coopération avec une partie de cette-dernière,
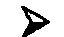 et un jeu de tuyau flexible pour perfusion, un tuyau (13) passant à travers une pince à tuyau flexible (1) avec laquelle le tuyau (13) peut être au choix ouvert et/ou verrouillé,
sont utilisés
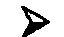 la pince à tuyau flexible (1) servant au choix à verrouiller et à ouvrir des tuyaux flexibles (13), par exemple des tuyaux flexibles pour perfusion, ayant la forme d'un arceau essentiellement en U avec des branches rigides (2, 3) sur les faces internes desquelles sont formées des saillies (4, 5) coopérant en formant des arrêtes de compression, avec une entretoise (6) flexible de façon élastique grâce à laquelle les branches (2, 3) peuvent être écartées en une position divergente, et avec une lame flexible de façon élastique pouvant être écartée, ladite lame étant reliée à l'extrémité libre de la première branche (2) et courbée en direction de la seconde branche (3), étant munie d'un bec d'enclenchement (8) s'étendant vers l'intérieur et derrière lequel l'extrémité libre de la deuxième branche (3) s'enclique en position verrouillée lorsque les branches (2, 3) sont pressées ensemble, l'entretoise (6) et la lame flexible (7) étant munies de trous (9, 10) pour laisser passer le tuyau flexible (13), et la pince à tuyau flexible (1) étant munie de moyens (11) pour fermer la pince à tuyau flexible (1) et/ou de moyens (16) pour ouvrir la pince à tuyau flexible (1) qui lorsqu'une pression est exercée par l'élément d'actionnement (12) d'une pompe pour perfusion respectivement ouvre et/ou ferme la pince à tuyau flexible (1).

2. Pince pour tuyau flexible destinée à être utilisée dans une combinaison selon la revendication 1, pour au choix verrouiller et ouvrir des tuyaux flexibles, par exemple des tuyaux flexibles pour perfusion, ayant la forme d'un arceau essentiellement en U avec des branches rigides sur les faces internes desquelles sont formées des saillies coopérant en formant des arrêtes de compression, avec une entretoise flexible de façon élastique grâce à laquelle les branches peuvent être écartées en une position divergente, et avec une lame flexible de façon élastique pouvant être écartée et étant reliée à l'extrémité libre de la première branche et courbée en direction de la seconde branche, et présentant un bec d'enclenchement s'étendant vers l'intérieur et derrière lequel l'extrémité libre de la deuxième branche s'enclique en position verrouillée lorsque les branches sont pressées ensemble, l'entretoise et la lame flexible étant munies de trous pour laisser passer le tuyau flexible, **caractérisée en ce que** la pince à tuyau flexible (1) est munie de moyens (11) pour fermer la pince à tuyau flexible et/ou de moyens (16) pour ouvrir la pince à tuyau flexible (1) qui lorsqu'une pression est exercée par un élément d'actionnement d'une pompe pour perfusion respectivement ouvre et/ou ferme la pince à tuyau flexible (1).

3. Pince pour tuyau flexible selon la revendication 2, **caractérisée en ce que** les moyens (11 ) pour fermer la pince à tuyau flexible (1) ont la forme d'un élément (11 ) sur la pince à tuyau flexible (1) sur lequel une partie de la pompe pour perfusion peut exercer une force agissant verticalement sur la deuxième branche (3) de la pince pour tuyau flexible (1 ).

4. Pince pour tuyau flexible selon la revendication 3, **caractérisée en ce que** les moyens (11 ) pour fermer la pince à tuyau flexible (1) ont la forme d'un bombement (11 ) formé sur la face extérieure de la deuxième branche (3), sur lequel peut rouler au moins une partie (14) d'un levier basculant (12) de la pompe pour perfusion.

5. Pince pour tuyau flexible selon la revendication 4, **caractérisée en ce que** les moyens (11) pour fermer la pince à tuyau flexible (1) sont munis de dentures qui peuvent coopérer avec des dentures correspondantes formées sur le levier basculant (12).

6. Pince à tuyau flexible selon l'une des revendications 2 à 5, **caractérisée en ce qu'**un tuyau souple (13) placé dans la pince à tuyau flexible (1) peut être fermé de façon étanche au liquide par l'action commune des moyens (11) pour fermer la pince à tuyau flexible (1) et du levier pivotant (12).

7. Pince à tuyau flexible selon l'une des revendications 2 à 5, **caractérisée en ce que** la pince à tuyau flexible (1) est encliquetable par l'action commune des moyens (11) pour fermer la pince à tuyau flexible (1) et du levier pivotant (12).

8. Pince à tuyau flexible selon la revendication 2, **caractérisée en ce que** les moyens (16) pour ouvrir la pince à tuyau flexible (1) ont la forme d'un élément (s16) placé sur la pince à tuyau flexible (1) au moyen duquel une partie de la pompe pour perfusion peut appuyer la lame flexible (7) en l'éloignant de la deuxième branche (3).

9. Pince pour tuyau flexible selon la revendication 8, **caractérisée en ce que** les moyens (16) pour ouvrir la pince à tuyau flexible (1) ont la forme d'un élément (16) pour coopérer avec au moins une partie (15) du levier pivotant (12) de la pompe pour perfusion, la lame flexible (7) étant éloignée de la deuxième branche (3).

10. Pompe pour perfusion destinée à être utilisée dans une combinaison selon la revendication 1, munie d'une porte pouvant être verrouillée et déverrouillée pour mettre en place ou enlever un tuyau flexible passant à travers une pince à tuyau flexible, la pince à tuyau flexible pouvant être au choix ouverte et/ou verrouillée ou bien être encliquetable en position verrouillée, **caractérisée en ce que** la pompe pour perfusion est munie pour fermer et/ou ouvrir une pince à tuyau flexible (1) selon les revendications 2 à 9 d'un élément d'actionnement (12) qui ouvre et/ou ferme la pince à tuyau flexible (1) lorsque une pression est exercée respectivement sur les moyens (11) pour fermer et/ou sur les moyens (16) pour ouvrir la pince à tuyau flexible (1 ).

11. Jeu de tuyau flexible, un tuyau flexible étant passé à travers une pince à tuyau flexible selon les revendications 2 à 9 avec laquelle le tuyau flexible peut être au choix ouvert et/ou fermé, destiné à être utilisé dans une pompe pour perfusion selon la revendication 10.
